(19) Europäisches Patentamt
European Patent Office
Office européen des brevets

(11) Veröffentlichungsnummer: **0 096 297**
**B1**

(12) # EUROPÄISCHE PATENTSCHRIFT

(45) Veröffentlichungstag der Patentschrift: **15.06.88**

(21) Anmeldenummer: **83105155.2**

(22) Anmeldetag: **25.05.83**

(51) Int. Cl.[4]: **C 07 D 417/12,**
C 07 D 413/12,
C 07 D 403/12,
C 07 D 277/40,
C 07 D 263/48, C 07 F 7/10,
A 61 K 31/41 // C07D205/08,
C07D407/04

(54) **Verfahren zur Herstellung von 1-Sulfo-2-oxoazetidinderivaten.**

(30) Priorität: **03.06.82 CH 3416/82**
**03.06.82 CH 3417/82**
**25.04.83 CH 2201/83**

(43) Veröffentlichungstag der Anmeldung:
**21.12.83 Patentblatt 83/51**

(45) Bekanntmachung des Hinweises auf die Patenterteilung:
**15.06.88 Patentblatt 88/24**

(84) Benannte Vertragsstaaten:
**AT BE CH DE FR GB IT LI LU NL SE**

(56) Entgegenhaltungen:
**EP-A-0 009 008**
**EP-A-0 021 678**
**EP-A-0 037 380**
**EP-A-0 053 816**
**EP-A-0 059 683**
**EP-A-0 075 104**
**EP-A-0 076 452**
**DE-A-3 239 157**
**GB-A-2 071 650**
**US-A-4 379 787**

(73) Patentinhaber: **F. HOFFMANN-LA ROCHE & CO. Aktiengesellschaft**
**CH-4002 Basel (CH)**

(72) Erfinder: **Furlenmeier, André, Dr.**
**Wettsteinallee 119**
**CH-4058 Basel (CH)**
Erfinder: **Hubschwerlen, Christian Nicolas, Dr.**
**Rue de la Gendarmerie**
**F-68200 Durmenach (FR)**
Erfinder: **Hofheinz, Werner, Dr.**
**Talmattweg 7**
**CH-4103 Bottmingen (CH)**
Erfinder: **Isenring, Hans-Peter, Dr.**
**Himmelrainweg 5**
**CH-4450 Sissach (CH)**

(74) Vertreter: **Lederer, Franz, Dr. et al**
**Van der Werth, Lederer & Riederer**
**Patentanwälte Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 096 297 B1

Courier Press, Leamington Spa, England.

**Beschreibung**

Die vorliegende Erfindung betrifft ein Verfahren zur Herstellung von Carbonsäuren der allgemeinen Formel

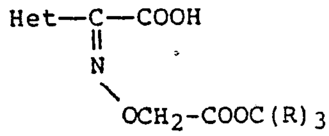

IV a

in der Het einen ggfs. aminosubstituierten 5- oder 6-gliedrigen, aromatischen, 1 oder 2 Stickstoffatome, ggfs, auch ein Sauerstoff- oder Schwefelatom enthaltenden Heterocyclus und R $C_{1-3}$-Alkyl bedeutet, und die Gruppe $=NOCH_2COOC(R)_3$ mindestens teilweise in syn-Form vorliegt, das dadurch gekennzeichnet ist, dass man in einem Ester der allgemeinen Formel

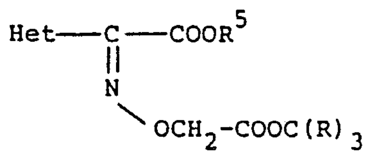

X

in der Het und R die oben gegebene Bedeutung haben und $R^5$ Allyl darstellt, und die Gruppe $=NOCH_2COOC(R)_3$ mindestens teilweise in syn-Form vorliegt,
die Allylgruppe $R^5$ mit Hilfe einer Palladiumverbindung in Gegenwart von Triphenylphosphin oder eines Tri(niederen Alkyl)phosphits und ferner in Gegenwart eines Alkalimetallalkanoats oder einer organischen Base katalytisch abspaltet.

Die vorliegende Erfindung betrifft auch ein Verfahren zur Herstellung von Benzthiazolthioestern der allgemeinen Formel

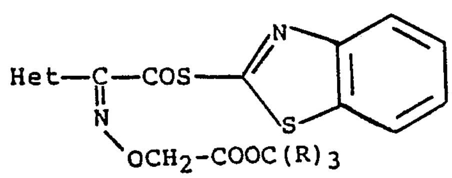

III

in der R und Het obige Bedeutung haben, und die Gruppe $=NOCH_2COOC(R)_3$ mindestens teilweise in syn-Form vorliegt,
das dadurch gekennzeichnet ist, dass man eine erfindungsgemäss erhaltene Carbonsaüre der obigen allgemeinen Formel IVa mit Dithio-bis-benzthiazol in Gegenwart eines Tri(niederen Alkyl)phosphits und einer Base oder in Gegenwart von Triphenylphosphin behandelt.

Die vorliegende Erfindung betrifft ferner ein Verfahren zur Herstellung von 1-Sulfo-2-oxoazetidinderivaten der allgemeinen Formel

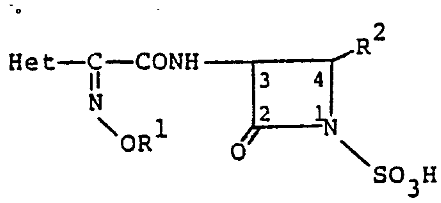

I

worin $R^1$ die Gruppe $—CH_2—COOC(R)_3$ oder Carboxymethyl und $R^2$ Wasserstoff, niederes Alkyl, niederes Alkenyl, niederes Alkinyl, niederes Alkoxycarbonyl, niederes Alkanoyloxy-niederes Alkyl, niederes Alkoxycarbonyl-niederes Alkenyl, Hydroxyiminomethyl, niederes Alkoxyiminomethyl, Carbamoyl, Carbamoyl-niederes Alkenyl oder Carbamoyloxy-niederes Alkyl bedeuten, und Het und R obige Bedeutung haben,
das dadurch gekennzeichnet ist, dass man einen erfindungsgemäss erhaltenen Benzthiazolthioester der obigen allgemeinen Formel III durch Umsetzen mit einer in racemischer Form oder in Form des 3S-Enantiomeren vorliegenden Verbindung der allgemeinen Formel

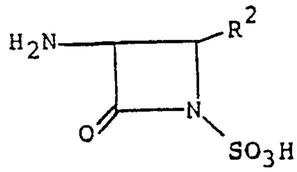

II

worin $R^2$ obige Bedeutung besitzt,
in eine in racemischer Form oder in Form des 3S-Enantiomeren vorliegende Verbindung der allgemeinen Formel

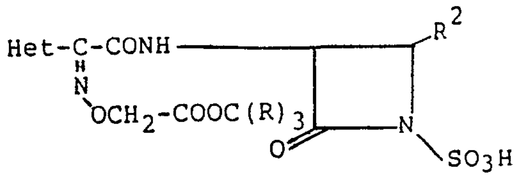

Ib

worin Het, R und $R^2$ obige Bedeutung besitzen, und die Gruppe $=NOCH_2COOC(R)_3$ mindestens teilweise in syn-Form vorliegt,
überführt, erwünschtenfalls die Gruppe $—CH_2—COOC(R)_3$ in Carboxymethyl umwandelt und erwünschtenfalls ein erhaltenes Produkt in ein pharmazeutisch verträgliches Salz überführt.

Der mit "Het" bezeichnete Heterocyclus schliesst alle 5- oder 6-gliedrigen, aromatischen Ringstrukturen ein, welche 1 oder 2 Stickstoffatome besitzen und ggfs. durch eine Aminogruppe substituiert sind, z.B. Pyrazolylgruppen, wie 2-Pyrazol-3-yl, Amino-pyridylgruppen, wie 2-Amino-6-pyridyl, Amino-imidazolylgruppen, wie 2-Amino-4-imidazolyl. Gegebenenfalls können sie ein Sauerstoffatom enthalten, wie z.B. in Amino-oxazolylgruppen, z.B. 2-Amino-4-oxazolyl oder ein Schwefelatom, wie z.B. in Amino-thiadiazolylgruppen, wie 5-Amino-3-(1,2,4-thiadiazolyl) oder, insbesondere, Aminothiazolylgruppen, wie 2-Amino-4-thiazolyl.

Der Ausdruck "niederes Alkyl" allein oder in Zusammensetzungen bedeutet eine aliphatisches Kohlenwasserstoffgruppe, welche geradkettig oder verzweigt sein kann und bevorzugt bis zu 7 Kohlenstoffatome enthält, wie z.B. Methyl, Aethyl n-Propyl, Isopropyl, n-Butyl, Isobutyl, sec-Butyl, t-Butyl, n-Pentyl, Isopentyl, n-Hexyl, n-Heptyl usw. Der Ausdruck "niederes Alkoxy" hat analoge Bedeutung. Der Ausdruck "niederes Alkenyl" allein oder in Zusammensetzungen bedeutet eine olefinische Kohlenwasserstoffgruppe, die geradkettig oder verzweigt sein kann und bevorzugt bis zu 7 Kohlenstoffatome enthält, wie z.B. Vinyl, Allyl, Isopropenyl, 2-Methallyl, 2-Butenyl, 3-Butenyl, 2-Hexenyl, 2-Heptenyl usw. Der Ausdruck "niederes Alkinyl" bedeutet eine acetylenische Kohlenwasserstoffgruppe, die geradkettig oder verzweigt sein kann und bevorzugt bis zu 7 Kohlenstoffatome enthält, wie z.B. Aethinyl, 1-Propinyl, 2-Propinyl, 2-Hexinyl, 2-Heptinyl usw. Der Ausdruck "niederes Alkanoyl" bzw. "niederes Alkanoyloxy" bedeutet einen aliphatischen Carbonsäurerest mit bevorzugt bis zu 7 Kohlenstoffatomen, wie z.B. Acetyl, Propionyl, Isobutyryl, Acetoxy, Propionyloxy, Isobutyryloxy.

R ist vorzugsweise Methyl.

Bevorzugte $R^1$-Gruppen sind: t-Butoxycarbonylmethyl und Carboxymethyl; ganz besonders Carboxymethyl.

Het ist vorzugsweise 2-Amino-4-thiazolyl.

Bevorzugte $R^2$-Gruppen sind Methyl, Aethyl, n-Propyl, Vinyl, Allyl, Aethinyl, 3-(Acetoxy)-n-propyl, Methoxycarbonyl, Hydroxyiminomethyl, Methoxyiminomethyl, 2-(Aethoxycarbonyl)-1-methylvinyl, Carbamoyl, Carbamoylvinyl und Carbamoyloxymethyl; ganz besonders Carbamoyl und Carbamoyloxymethyl.

Eine bevorzugte Gruppen der Formel

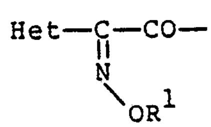

ist 2-(2-Amino-4-thiazolyl)-2-(carboxymethoxyimino)acetyl.

Die Verbindungen der Formel I können in verschiedenen isomeren Formen vorliegen [z.b. cis, trans; syn(Z-Form), anti(E-form); sowie als 3S-Enantiomere]. Gleich verhält es sich mit den nachstehend erläuterten Ausgangsverbindungen.

Die Verbindungen der Formel I können als freie Säuren bzw. als Betaine vorliegen oder auch als pharmazeutisch verträgliche Salze, welche durch Salzbildung an der freien 1-Sulfogruppe bzw. an einer allfälligen Carboxygruppe im Substituenten in 3-Stellung mit einem basischen Salzbildner erhalten werden. Als basische Salzbildner kommen z.B. in Betracht: anorganische Kationen, wie Natrium- und

Kaliumjonen, basische Aminosäuren, wie Arginin, Ornithin, Lysin oder Histidin, Polyhydroxyalkylamine, wie N-Methylglucamin, Diäthanolamin, Triäthanolamin usw.

Vorhandene Carboxygruppen in einer Verbindung der Formel I bzw. Salzen davon können durch entsprechende Veresterung in leicht hydrolysierbare Estergruppen übergeführt werden. Solche leicht hydrolysierbaren Estergruppen, welche im Körper in die entsprechenden freien Carboxygruppen gespalten werden, sind z.B. α-(Niederalkoxy)-nieder-alkoxy-carbonylgruppen, wie Methoxymethoxycarbonyl, α-Methoxyäthoxycarbonyl, niedere Alkylthiomethoxycarbonylgruppen, wie Methylthiomethoxycarbonyl, α-(Niederalkanoyl)-nieder-alkoxycarbonyl, wie Acetoxymethoxycarbonyl, Pivaloyloxymethoxycarbonyl, α-Pivaloyloxyäthoxycarbonyl; α-(Niederalkoxycarbonyl)-nieder-alkoxycarbonyl, wie Aethoxycarbonyloxy-methoxycarbonyl, t-Butoxycarbonylmethoxycarbonyl, oder α-Aethoxycarbonyloxycarbonyl; Lactonyl-gruppen, wie Phthalidyl oder Thiophthalidyl; oder die Gruppe

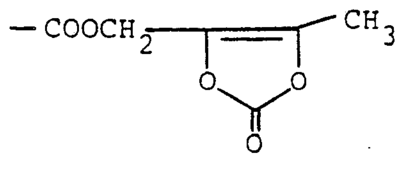

Beispiele von Verbindungen der Formel I, welche erfindungsgemäss hergestellt werden können, sind die in den nachstehenden Beispielen 1a und 1b beschriebenen Endprodukte sowohl in der Form wie sie gemäss den Beispielen vorliegen (3S-Enantiomere bzw. Racemate) sowie auch in Form von leicht hydrolysierbaren Estern und pharmazeutisch verträglichen Salzen dieser Verbindungen.

Besonders bevorzugte Verbindungen unter der Formel I sind die Verbindungen der allgemeinen Formel

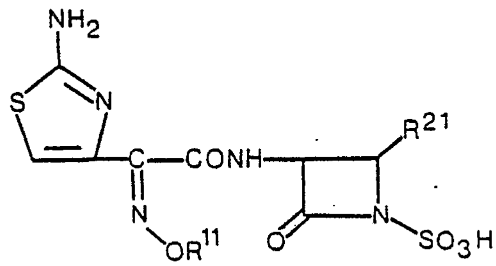

Ia

worin $R^{11}$ Carboxymethyl und $R^{21}$ Carbamoyl oder Carbamoyloxymethyl darstellt, in racemischer Form oder in Form der 3S-Enantiomeren sowie die entsprechenden leicht hydrolysierbaren Ester und pharmazeutisch verträgliche Salze dieser Verbindungen.

Besonders bevorzugt unter diesen Verbindungen ist die (3S,4S)-3-[(Z)-2-(2-Amino-4-thiazolyl)-2-[(carboxymethoxy)imino]acetamido]-4-carbamoyloxymethyl-2-oxo-1-azetidin-sulfonsäure sowie deren pharmazeutisch verträgliche Salze.

Die erfindungsgemässe Umsetzung der Verbindungen der allgemeinen Formeln II und III wird zweckmässig in einem inerten organischen Lösungsmittel durchgeführt. z.B. in einem chlorierten Kohlenwasserstoff, wie Methylenchlorid oder Chloroform, in einem Aether, z.B. Tetrahydrofuran oder Dioxan, in einem Ester, z.B. Aethylacetat, in einem Keton, z.B. Aceton, in einem aprotischen Lösungsmittel, wie Acetonitril, Dimethylformamid oder Dimethylacetamid, oder in einem Gemisch eines dieser Lösungsmittel mit Wasser. Die Reaktionstemperatur liegt zweckmässig zwischen etwa −40° und +60°C, vorteilhaft zwischen −15° und +25°C, insbesondere 0—20°C. Die Reaktionsteilnehmer werden zweckmässig in etwa stöchiometrischem Verhältnis verwendet oder mit einem leichten Ueberschuss an Thioester der Formel III. Die Reaktion wird vorteilhaft in Gegenwart einer Base durchgeführt, wie z.B. eines organischen Amins, wie Thiäthylamin oder N-Methylmorpholin, oder eines Alkalimetallbicarbonats, wie Natriumbicarbonat.

Die im Reaktionsprodukt der Formel Ib vorhandene Gruppe der Formel $—CH_2COOC(R)_3$ kann erwünschtenfalls in die entsprechende Carboxymethylgruppe umgewandelt werden, und zwar durch Behandeln mit einer starken Säure, wie Trifluoressigsäure (ggfs. in Gegenwart von Anisol), Salzsäure oder p-Toluolsulfonsäure, bei niederer Temperatur, wie −10°C bei Raumtemperatur.

Die Herstellung der Salze der Verfahrensprodukte der Formel I kann in an sich bekannter Weise erfolgen, z.B. durch Umsetzung einer Säure der Formel I mit einer äquivalenten Menge der gewünschten Base, ggfs. in Form eines Ionenaustauschers. Man arbeitet zweckmässig in einem Lösungsmittel, wie Wasser, oder in einem organischen Lösungsmittel, wie Aethanol, Methanol, Aceton, Aethylacetat und dgl. Die Temperatur der Salzbildung ist nicht kritisch, sie liegt aber im allgemeinen im Bereich von etwa 0—50°C, vorzugsweise bei Raumtemperatur.

Die Thioester der Formel III werden, wie erwähnt, dadurch hergestellt, dass man eine erfindungsgemäss erhaltene Carbonsäure der obigen allgemeinen Formel IVa mit Dithio-bis-benzthiazol in

Gegenwart eines Tri-(niederalkyl)-phosphits und einer Base oder in Gegenwart von Tri-phenyl-phosphin umsetzt. Die Reaktionstemperatur liegt zweckmässig zwischen etwa −30° und +50°C, vorteilhaft zwischen etwa −20° und +25°C. Die Veresterung wird vorteilhaft in einem organischen Lösungsmittel durchgefuhrt, z.B. in Acetonitril oder in Methylenchlorid. Die bevorzugte Ausführungsform ist die Umsetzung in Gegenwart eines Tri-(nieder-alkyl)-phosphits und einer Base. Als Tri-(nieder-alkyl)-phosphit wird vorzugsweise Triäthylphosphit und als Base vorzugsweise eine organische Base, insbesondere eine tertiäre organische Base, wie Triäthylamin, N-Aethyl-diisopropylamin oder, vorzugsweise, N-Methylmorpholin, verwendet.

Ein besonders bevorzugter Thioester der Formel III ist der 2-(2-Amino-4-thiazolyl)-2-[[(Z)-1-(t-butoxycarbonyl)-methoxy]-imino]-essigsäure-2-benzthiazolyl-thioester.

Ein besonderes Problem stellt sich bei der Herstellung der Säuren der Formel IVa.

Der herkömmliche Weg zur Gewinnung solcher Verbindungen, nämlich Umsetzung des Methyl- oder Aethylesters der entsprechenden Hydroxyiminoverbindung mit einem Halogen-essigsäure-$C(R)_3$-ester und anschliessende Verseifung liefert aber nicht die gewünschte Säure der Formel IVa denn die Gruppe $-COOC(R)_3$ wird verseift. Es ist somit notwendig, einen anderen Syntheseweg einzuschlagen.

Die erwähnte Säure der Formel IVa kann aber in guter Ausbeute gewonnen werden, wenn man anstelle der erwähnten Methyl- oder Aethylester den Allylester einsetzt, d.h. dass man in einem Ester der allgemeinen Formel

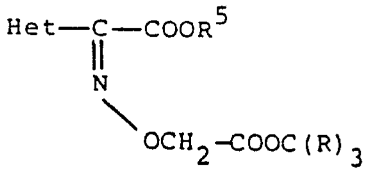

X

in der Het, R und $R^5$ die oben angegebene Bedeutung haben, die Allylsgruppe $R^5$ abspaltet.

"$C_{1-3}$-Alkyl" schliesst ein: Methyl, Aethyl, n-Propyl und Isopropyl. Der bevorzugte Rest der Formel $-COO-C(R)_3$, ist derjenige, worin R Methyl darstellt, d.h. t-Butoxy-carbonyl.

Die obige Spaltung erfolgt durch Einwirken einer Palladiumverbindung in Gegenwart von Triphenylphosphin oder eines Tri-(niederalkyl)-phosphits, vorzugsweise Triäthylphosphit. Als Palladiumverbindungen kommen in Betracht: Palladiumkohle, Palladiumsalze, insbesondere Salze mit Halogenwasserstoffsäuren, wie Chlor- oder Bromwasserstoffsaüre, oder mit niederen Alkancarbonsäuren, wie Essigsäure oder Propionsäure. Auch palladiumorganische Komplexe mit Triphenylphosphin oder einem Tri-(niederalkyl)-phosphit, wie Triäthylphosphit, kommen in Frage, wobei auch ohne zusätzliches Triphenylphosphin oder Tri-(niederalkyl)-phosphit gearbeitet werden kann. Als Palladiumverbindung verwendet man vorzugsweise Palladiumkohle, Palladiumchlorid oder Palladiumacetat. Weiterer Reaktionsteilnehmer ist ein Alkalimetallalkanoat, z.B. Natriumacetat oder, vorzugsweise, Natrium-2-äthylcaproat, oder vorzugsweise eine organische Base, insbesondere Triathylamin oder N-Methylmorpholin. Die Reaktionstemperatur kann zwischen etwa 0 und 100°C schwanken, sie liegt aber vorzugsweise bei Zimmertemperatur (bei Verwendung von Palladiumkohle aber etwas höher; etwa 50°—80°C). Vorzugsweise wird in einem inerten organischen Lösungsmittel gearbeitet, z.B. in Aethylacetat oder Methylenchlorid.

Die Allylester der Formel X können hergestellt werden ausgehend von Diketen, Chlorgas und Allylalkohol, welche in Allyl-4-chloracetoacetat übergehen. Letzteres wird mit salpetriger Säure nitrosiert und anschliessend mit Thioharnstoff in den Het-2-(Z)-hydroxy-imino-essigsäure-allylester übergeführt, der hierauf mit einer Verbindung der allgemeinen Formel

$$Hal-CH_2-COO-C(R)_3 \qquad XI$$

in der R die obige Bedeutung hat und Hal Chlor, Brom oder Jod darstellt, in Gegenwart einer Base, wie Alkalicarbonat, Triäthylamin oder N-Aethyl-diisopropylamin, in den Allylester der Formel X umgewandelt wird.

Die Ausgangsverbindungen der Formel II können nach verschiedenen Methoden erhalten werden. Für die Herstellung von optisch einheitlichen Verbindungen der Formel II mit 3S-cis-Konfiguration kann man ausgehend von Isopropyliden-L-glyceraldehyd gemäss den nachstehenden Formelschemata arbeiten (Schemata I—VII). Die Herstellung von optisch einheitlichen Verbindungen der Formel II mit 3S-trans-Konfiguration wird in den Schemata V and VI veranschaulicht.

Schema I

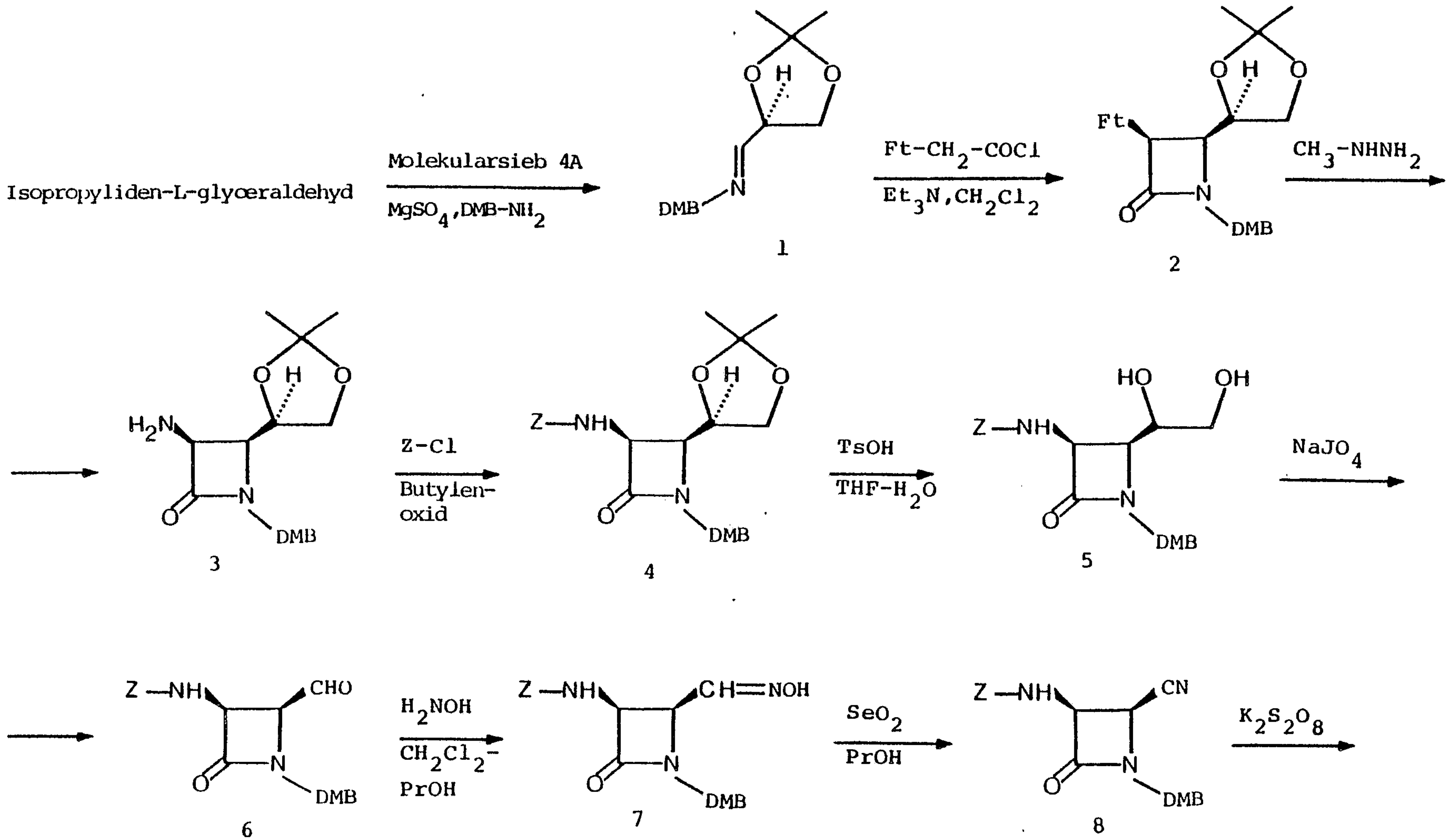


1 2 3 4 5 6 7 8

Schema 'I Forts.

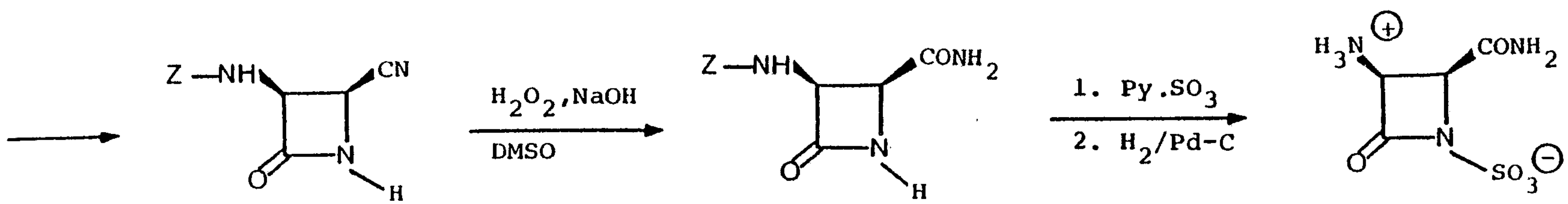


9 10 11

Schema II

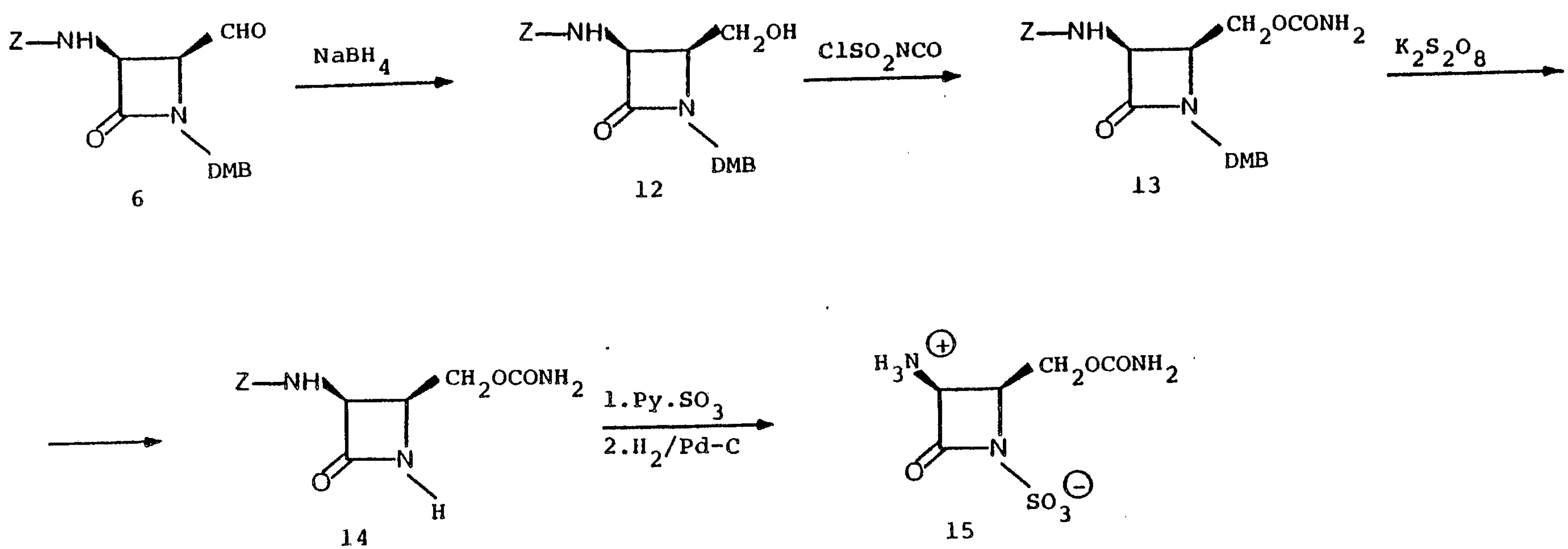


6 12 13 14 15

Schema III

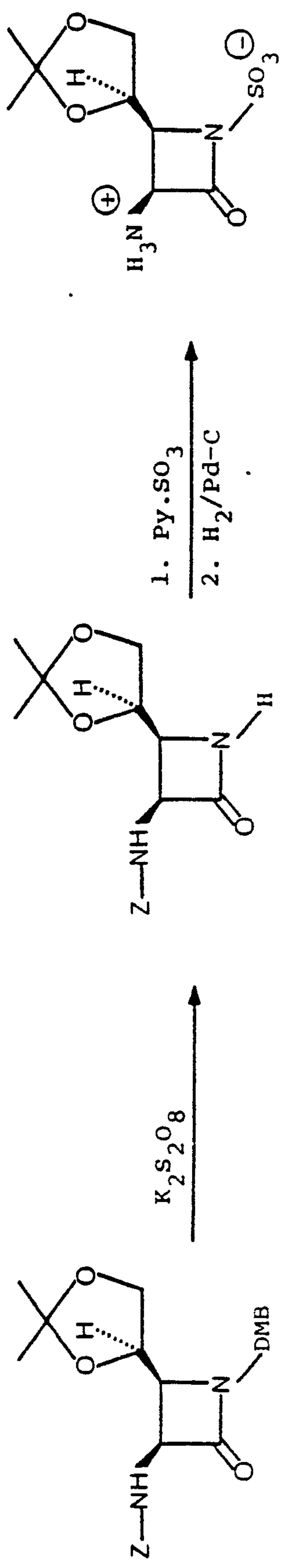


4

16

17

Schema IV

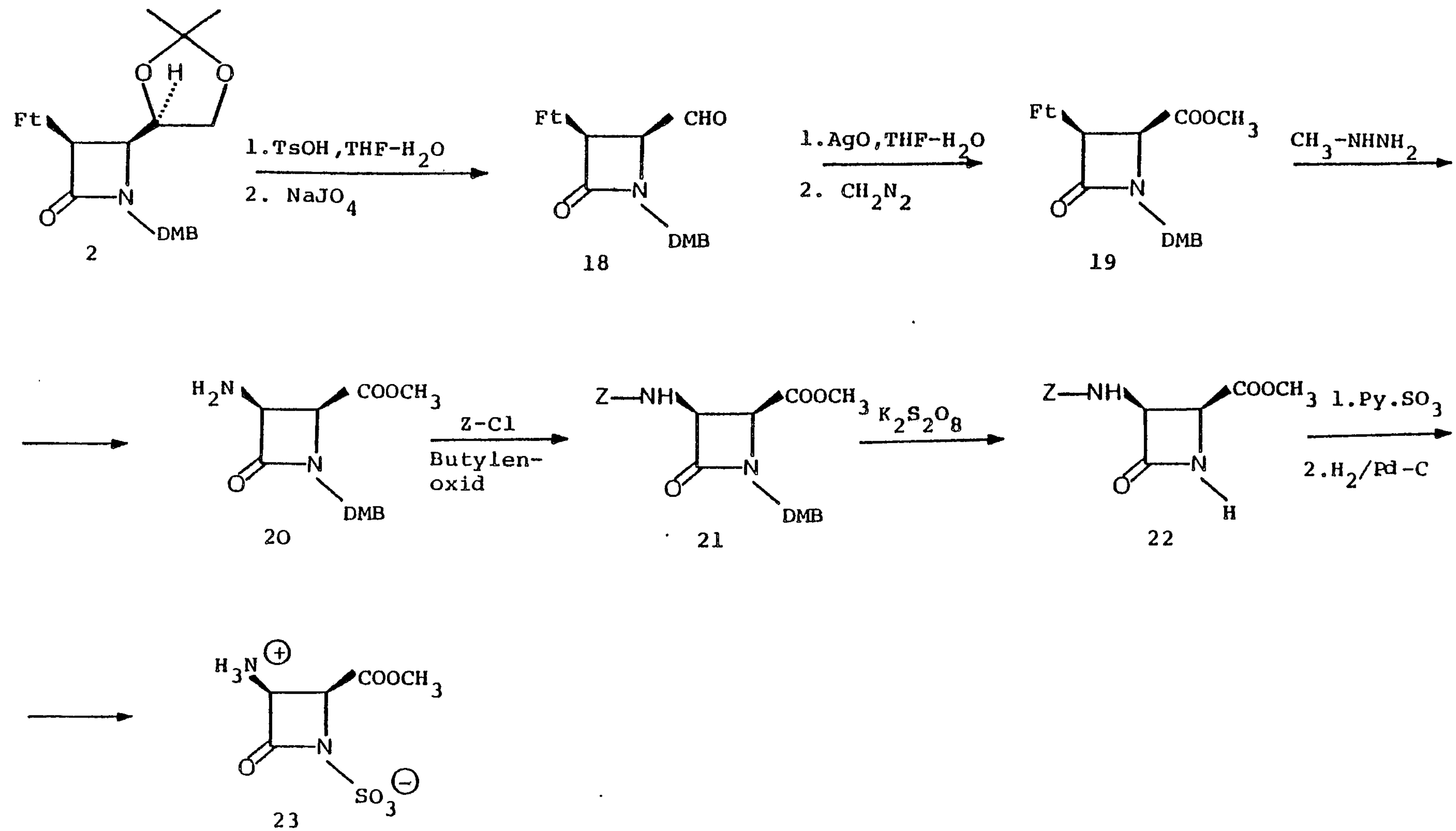


2 18 19 20 21 22 23

Schema V

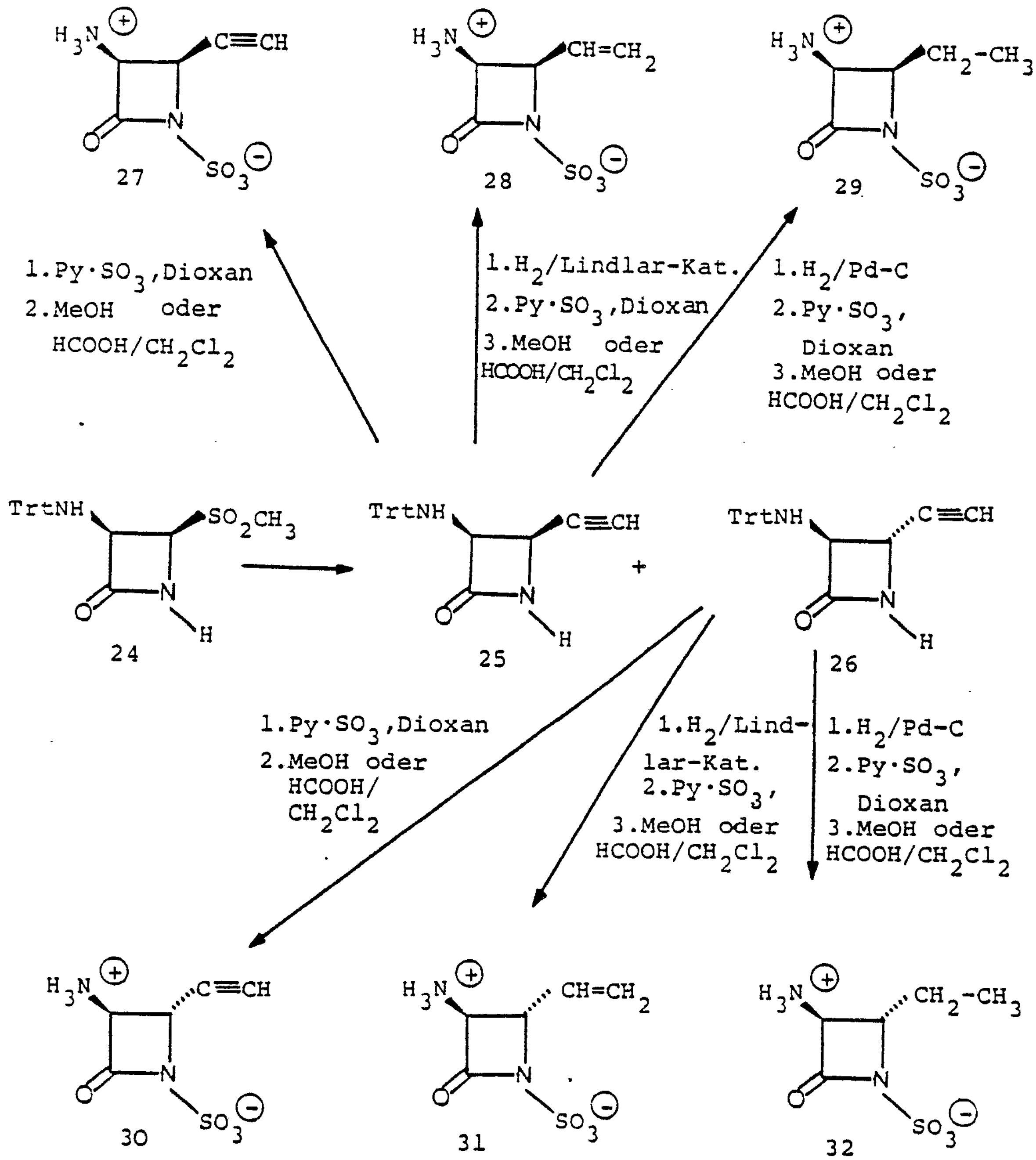


27 28 29

24 25 + 26

30 31 32

Schema VI

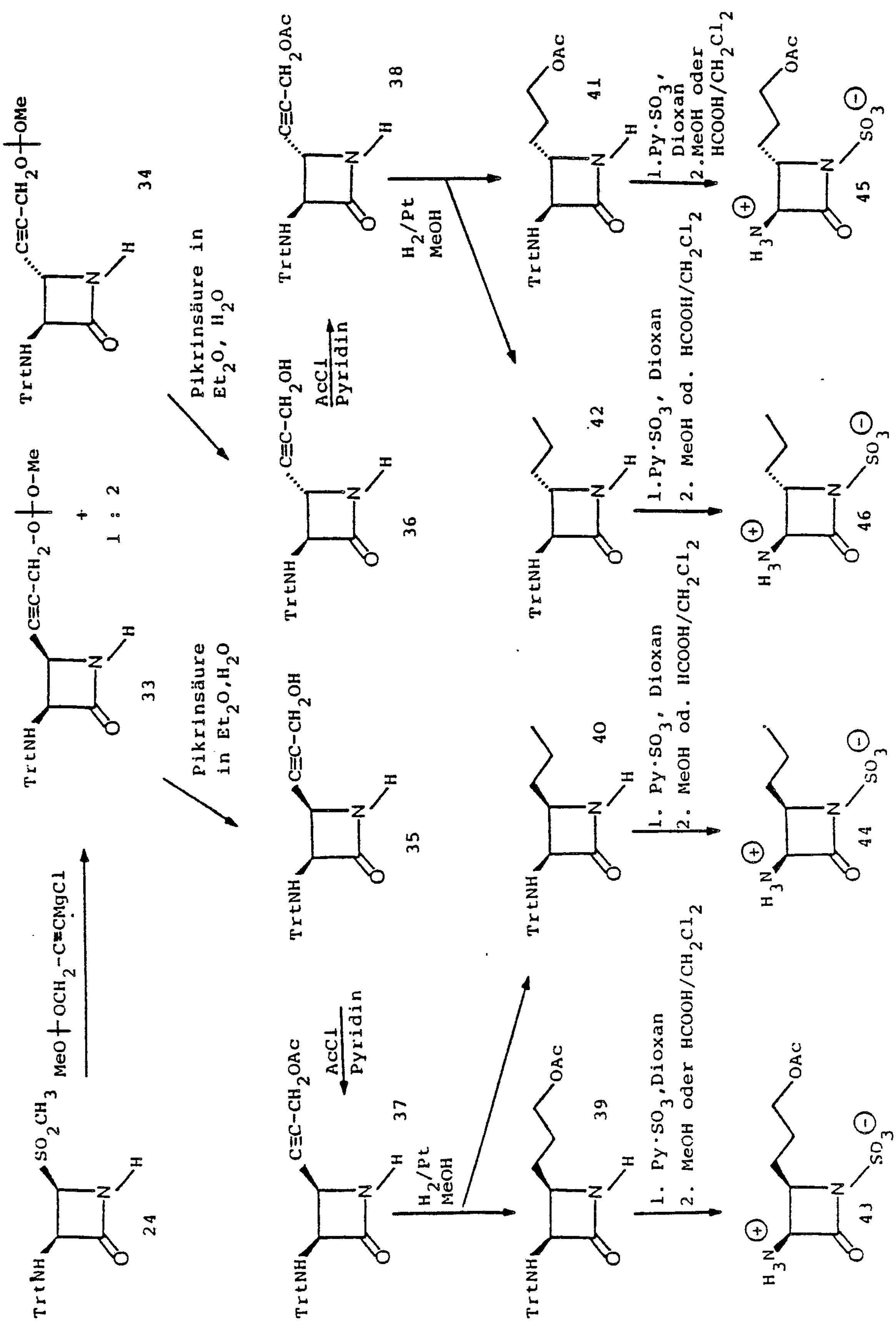


24, 33, 34, 35, 36, 37, 38, 39, 40, 41, 42, 43, 44, 45, 46

Schema VII

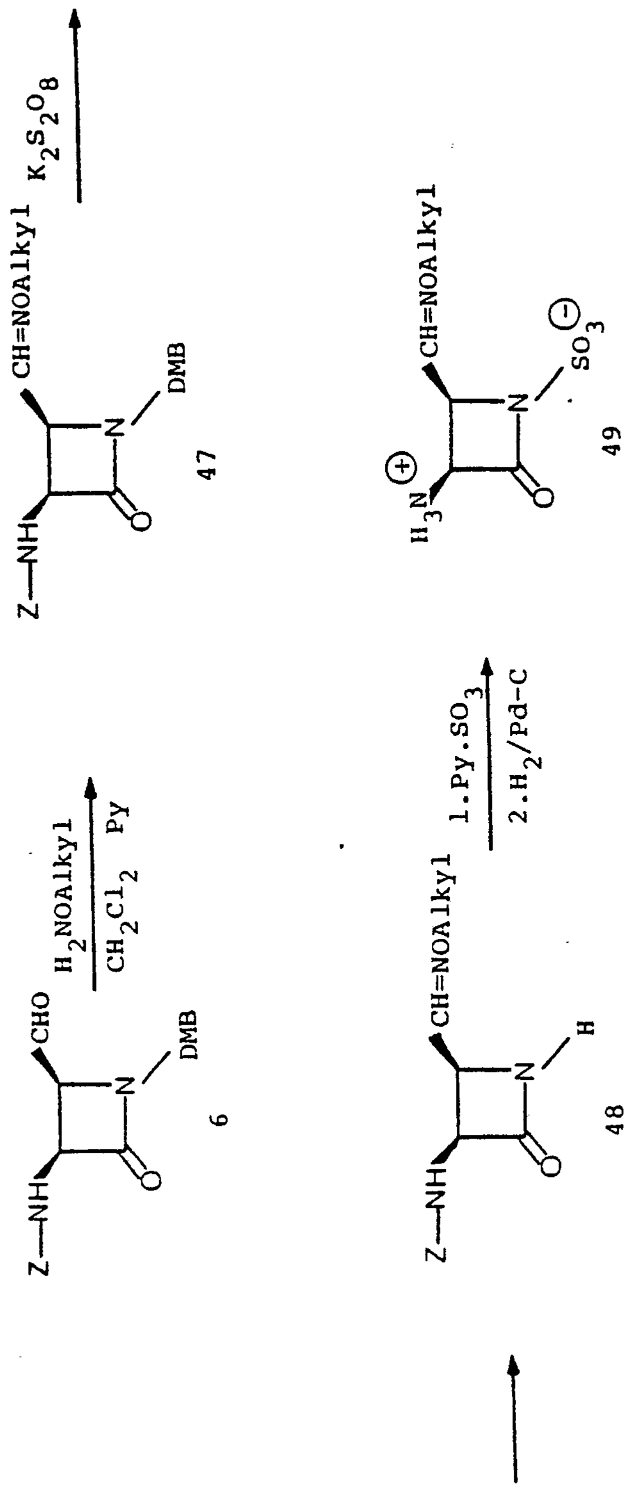


6

47

48

49

Erklärungen zu den Schemata I—VII

DMB = 2,4-Dimethoxybenzyl
Ft = Phthalimido
Et = Aethyl
Me = Methyl
TSOH = p-Toluolsulfonsäure
THF = Tetrahydrofuran
PrOH = n-Propanol
DMSO = Dimethylsulfoxid
Py = Pyridin
Py·$SO_3$ = Schwefeltrioxid-pyridinkomplex
Z = Benzyloxycarbonyl
Trt = Trityl
Ac = niederes Alkanoyl, z.B. Acetyl

Beispiel 1

a) 71,8 g (0,3 Mol) (3S,4S)-3-Amino-4-carbamoyloxymethyl-2-oxo-1-azetidinsulfonsäure werden in 1,5 l Methylenchlorid dispergiert und mit 45,6 g (0,45 Mol) Triäthylamin und 148,6 g (0,33 Mol) 2-(2-Amino-4-thiazolyl)-2-[[(Z)-(t-butoxycarbonyl)-methoxy]-imino]-essigsäure-2-benzthiazolylthioester unter Rühren versetzt. Das Reaktionsgemisch wird 5 Stunden bei Zimmertemperatur gerührt. Anschliessend setzt man 1,5 l Wasser zu, trennt die wässrige Phase, extrahiert zweimal mit 250 ml Methylenchlorid und säuert durch Zugabe von 850 ml 37%iger wässriger Salzsäure an. Nach 2-stündigem Rühren bei Zimmertemperatur wird die erhaltene Suspension auf 0°C abgekühlt und eine weitere 1/2 Stunde gerührt. Der Niederschlag wird abfiltriert, nacheinander mit 1000 ml kaltem Wasser, 1000 ml Methanol und 1000 ml Aether gewaschen und 12 Stunden bei 40°C/10 mm Hg getrocknet. Man erhält 111 g (79,3%) rohes (3S,4S)-3-[(Z)-2-(2-Amino-4-thiazolyl)-2-[(carboxymethoxy)-imino]-acetamido]-4-carbamoyloxymethyl-2-oxo-1-azetidinsulfonsaüre vom Schmelzpunkt 207°C, $[\alpha]_D^{20} = -46,2°$ (c = 1 in Dimethylsulfoxid).

Elementaranalyse berechnet für $C_{12}N_{14}N_6O_{10}S_2$
C 30,90, H 3,03, N 18,02, S 13,75%
gefunden
C 28,23, H 3,81, N 16,18, S 12,26, $H_2O$ 3,61%.
korrigiert für wasserfreie Substanz
C 29,29 H 3,53 N 16,79 S 12,72%
Eine analytische Probe ergibt die folgenden Daten:
$[\alpha]_D^{20} = +39°$ (c = 1 in Wasser); korrigiert für wasserfreie Substanz +42,5° (c = 0,9 in Wasser)
$[\alpha]_D^{20} = -43,5°$ (c = 1 in Dimethylsulfoxid); korrigiert für wasserfreie Substanz −47,4° (c = 0,9 in Wasser).
IR (KBr, $cm^{-1}$):
3458, 3428, 3354, 3291, 1777, 1712, 1648, 1617, 1557, 1531
$^1H$—NMR (DMSO, ppm):
3,9—4,4 (3H, m, CH—$CH_2$—O), 4,79 (2H, s, O—$\underline{CH_2}$—COOH), 5,30 (1H, dd, 5 und 9 Hz, NH—$\underline{CH}$—CH); 6,5 (6H, br., $NH_3^+$, $NH_2$, COOH), 6,90 (1H, s, H-Thiazol), 9,45 (1H, d, 9 Hz, CO—NH).
Elementaranalyse berechnet für $C_{12}H_{14}N_6O_{10}S_2$:
C 30,90, H 3,03 N 18,02, S 13,75%
gefunden
C 28,39, H 3,43, N 16,44, S 12,47% $H_2O$ 8,14%
korrigiert für wasserfreie Substanz:
C 30,91 H 2,74, N 17,90, S 13,58%.

b) 110 g (0,235 Mol) rohe (3S,4S)-3-[(Z)-2-(2-Amino-4-thiazolyl)-2-[(carboxymethoxy)-imino]-acetamido]-4-carbamoyloxymethyl-2-oxo-1-azetidinsulfonsäure, erhalten gemäss Beispiel 41, werden in 4,7 l Methanol dispergiert und mit 72,3 ml (0,518 Mol) Triäthylamin und 282,9 ml (0,566 Mol) einer 2N Lösung von Natrium-2-äthylcaproat in Aethylacetat behandelt. Die erhaltene Lösung wird 10 Minuten gerührt, mit 9 l Aceton verdünnt und auf 3 l konzentriert. Die zurückbleibende Suspension wird mit 3 l Aceton verdünnt, filtriert und das kristalline Salz mit Aether gewaschen und getrocknet. Man erhält 108,59 g (90,5%) (3S,4S)-3-[(Z)-2-(2-Amino-4-thiazolyl)-2-[(carboxymethoxy)-imino]-acetamido]-4-carbamoyloxymethyl-2-oxo-1-azetidinsulfonsäure-Dinatriumsalz. Dieses rohe Salz wird in 420 ml Wasser gelöst. 1050 ml Aethanol werden hinzugefügt und anschliessend (nach einigen Minuten) weitere 210 ml Aethanol. Die Lösung wird trübe und wird 1 Stunde gerührt. 1770 ml Aethanol werden nun tropfenweise innerhalb 2 Stunden hinzugegeben. Nach weiterem einstündigen Rühren und Abkühlen auf 0°C wird der Niederschlag abfiltriert und mit Aethanol gewaschen. Die erhaltenen Kristalle werden in 170 ml Aethanol und 680 ml Aether dispergiert, abfiltriert und mit Aether gewaschen. Nach dem Trocknen unter stark vermindertem Druck und 40°C erhält man 102,9 g (85,5%) (3S,4S)-3-[(Z)-2-(2-Amino-4-thiazolyl)-2-[(carboxymethoxy)-imino]-acetamido]-4-carbamoyloxymethyl-2-oxo-1-azetidinsulfonsäure-Dinatriumsalz.

Elementaranalyse berechnet für
$C_{12}H_{12}N_6O_{10}S_2Na_2$:
C 28,24, H 2,37, N 16,47, S 12,56%
gefunden
C 28,18, H 2,63, N 16,34, S 12,21, $H_2O$ 1,02%
korrigiert für wasserfreie Substanz:
C 28,46, H 2,65, N 16,50, S 12,33%

$[\alpha]_D^{20} = +19°$ (c = in Wasser)
IR (KBr, $cm^{-1}$): 1777, 1712, 1648, 1617, 1557, 1417
UV ($H_2O$; λ max (ε)): 295 nm (6850), 233 nm (12330)
$^1$H—NMR ($D_2O$, ppm): 4,2—4,8 (5H, m, CH—$CH_2$—O—CO, O—$CH_2$—COONa), 5,6 (1H, d, 5,5 Hz, O=C—CH), 7,05 (1H, s, H-Thiazol).

Der als Ausgangsmaterial verwendete 2 - (2 - Amino - 4 - thiazolyl) - 2 - [[(Z) - (t - butoxycarbonyl)methoxy]imino] - essigsäure - 2 - benzthiazolyl - thioester kann wie folgt hergestellt werden:

A. 84 g Diketen werden in 250 ml Tetrachlorkohlenstoff gelöst und auf −27°C gekühlt. Die Lösung wird trüb. 71 g Chlorgas werden innerhalb 5 Stunden unter Rühren langsam eingeleitet, wobei die Temperatur durch Kühlen bei −20 bis −30°C gehalten wird. Die erhaltene klare Lösung wird langsam innerhalb 1 Stunde zu einer Lösung von 58 g Allylalkohol in 250 ml Tetrachlorkohlenstoff und 80,5 ml Pyridin unter Rühren bei 0 bis −5°C gegeben. Nach zusätzlichem 15-minütigem Rühren ohne Kühlung wird das ausgefällte Pyridinhydrochlorid abfiltriert und mit 100 ml Tetrachlorkohlenstoff gewaschen. Die Tetrachlorkohlenstofflösung wird mit 2 × 300 ml Wasser gewaschen, über Natriumsulfat getrocknet und unter vermindertem Druck eingedampft. Der Rückstand wird destilliert; man erhält 136 g (77%) Allyl-4-chlor-acetoacetat als farblose Flüssigkeit von Siedepunkt 61—69°C (0,1 mm Hg).

Eine Lösung von 35,2 g Allyl-4-chlor-acetoacetat in 34 ml Essigsäure wird tropfenweise mit einer Lösung von 14,6 g Natriumnitrit in 21 ml Wasser innerhalb 45 Minuten unter Rühren und Kühlen versetzt. Während der Zugabe fällt die Temperatur allmählich von 9 auf −15°C. Anschliessend wird 2 Stunden bei −15°C gerührt. Eine auf 30°C vorgewärmte Lösung von 15,2 g Thioharnstoff in 120 ml Wasser wird mit der erhaltenen Lösung bei einer derartigen Geschwindigkeit versetzt, dass die Reaktionstemperatur bei etwa 30—35°C bleibt. Nach zusätzlichem 7-stündigem Rühren wird der erhaltene kristalline Niederschlag abfiltriert, nacheinander mit Wasser, Acetonitril und Aether gewaschen und aus Acetonitril kristallisiert. Man erhält 21,8 g (48%) 2-(2-Amino-4-thiazolyl)-2-(Z)-hydroxyimino-essigsäure-allylester vom Schmelzpunkt 184—185°C.

Eine Lösung von 20,4 g 2-(2-Amino-4-thiazolyl)-2-(Z)-hydroxyimino-essigsäure-allylester in 100 ml Dimethylsulfoxid und 100 ml Aceton werden zusammen mit 30 g Kaliumcarbonat und 19,5 ml t-Butylbromacetat 5 Stunden bei Zimmertemperatur gerührt. Nach Abdampfen des Acetons unter vermindertem Druck (Badtemperatur 50°C) werden 600 ml Aethylacetat hinzugefügt und die Lösung bis zur neutralen Reaktion mit Eiswasser gewaschen, getrocknet und unter vermindertem Druck eingedampft. Man erhält einen kristallinen, gelblichen Rückstand, der in Diisopropyläther dispergiert, filtriert und unter vermindertem Druck getrocknet wird. Man erhält 20,1 g (65,5%) 2 - (2 - Amino - 4 - thiazolyl) - 2 - [[(Z) - (t - butoxycarbonyl) - methoxy] - imino] - essigsäure - allylester vom Schmelzpunkt 135—136°C.

19 g 2 - (2 - Amino - 4 - thiazolyl) - 2 - [[(Z) - (t - butoxycarbonyl) - methoxy] - imino] - essigsäure - allylester in 500 ml Aethylacetat werden unter Rühren nacheinander mit 0,09 g Palladiumchlorid, 0,46 ml Triäthylphosphit und 30 ml Natrium-2-äthylcaproatlösung (2N Lösung in Aethylacetat) versetzt. Nach vierstündigem Rühren bei Zimmertemperatur werden 500 ml Wasser und 100 ml gesättigter, wässriger Natriumbicarbonatlösung hinzugefügt. Die wässrige Lösung wird abgetrennt, mit 100 ml Aethylacetat gewaschen und mit 2N wässriger Salzsäure auf pH 2 angesäuert. Der kristalline Niederschlag wird abfiltriert, nacheinander mit Wasser, Acetonitril und Aether gewaschen und unter vermindertem Druck getrocknet. Man erhält 14,6 g (87%) 2 - (2 - Amino - 4 - thiazolyl) - 2 - [[(Z) - (t - butoxycarbonyl) - methoxy] - imino] - essigsäure von Schmelzpunkt 175—176°C (Zers.).

Weitere Methoden zur Herstellung des Ausgangsmaterials sind die folgenden:

B. Der 2 - (2 - Amino - 4 - thiazolyl) - 2 - [[(Z) - (t - butoxycarbonyl) - methoxy] - imino] - essigsäure - allylester wird gemäss A oben umgesetzt, jedoch unter Verwendung von 0,12 g Palladiumacetat anstelle von Palladiumchlorid. Nach identischer Aufarbeitung erhält man 14,5 g (86,3%) 2 - (2 - Amino - 4 - thiazolyl) - 2 - [[(Z) - (t - butoxycarbonyl) - methoxy] - imino] - essigsäure vom Schmelzpunkt 171—172°C.

C. Der 2 - (2 - Amino - 4 - thiazolyl) - 2 - [[(Z) - (t - butoxycarbonyl) - methoxy] - imino] - essigsäure - allylester wird analog B oben umgesetzt, jedoch unter Verwendung von 0,695 g Triphenylphosphin anstelle von Triäthylphosphit. Man erhält 3,4 g (79,7%) 2 - (2 - Amino - 4 - thiazolyl) - 2 - [[(Z) - (t - butoxycarbonyl) - methoxy] - imino] - essigsäure vom Schmelzpunkt 167—169°C.

D. Eine Suspension von 1,4 g 2 - (2 - Amino - 4 - thiazolyl) - 2 - [[(Z) - (t - butoxycarbonyl) - methoxy] - imino] - essigsäure - allylester, 86 mg Palladiumkohle (5%), 0,033 ml Triäthylphosphit und 2,25 ml Natrium-2-äthylcaproatlösung (2N Lösung in Aethylacetat) in 50 ml Aethylacetat werden 12 Stunden bei 60°C gerührt. Nach dem Erkalten auf Zimmertemperatur wird das Reaktionsgemisch gemäss

C. oben aufgearbeitet. Man erhält 0,81 (65,5%) 2 - (2 - Amino - 4 - thiazolyl) - 2 - [[(Z) - (t - butoxycarbonyl) - methoxy] - imino] - essigsäure vom Schmelzpunkt 174—175°C.

E. 3,41 g 2 - (2 - Amino - 4 - thiazolyl) - 2 - [[(Z) - (t - butoxycarbonyl) - methoxy] - imino] - essigsäure - allylester werden in 100 ml Aethylacetat dispergiert und mit 250 mg Triphenylphosphin und 250 mg Tetrakis - (triphenylphosphin) - palladium versetzt. Die erhaltene Lösung wird mit 5,5 ml Natrium - 2 - äthylcaproatlösung (2N Lösung in Aethylacetat) versetzt. In kurzer Zeit entsteht ein dicker, unrührbarer Brei. Nach 15-minütigem Stehen bei Zimmertemperatur wird dieser einmal mit 100 ml Wasser und einmal mit 30 ml gesättiger wässriger Natriumcarbonatlösung geschüttelt; die wässrigen Lösungen werden vereinigt und einmal mit 50 ml Aethylacetat gewaschen. Die vereinigten wässrigen Lösungen werden mit 2N wässriger Salzsäure auf pH 2 gestellt. Der kristalline Niederschlag wird abfiltriert und nacheinander mit Wasser, Acetonitril und Aether gewaschen. Man erhält 2,4 g 2 - (2 - Amino - 4 - thiazolyl) - 2 - [[(Z) - (t - butoxycarbonyl) - methoxy] - imino] - essigsäure von Schmelzpunkt 167—169°C (Zers.).

F. 3,41 g 2 - (2 - Amino - 4 - thiazolyl) - 2 - [[(Z) - (t - butoxycarbonyl)methoxy] - imino] - essigsäure - allylester werden in 100 ml Aethylacetat dispergiert und mit 18 mg Palladiumchlorid und 0,084 ml Triäthylphosphit versetzt. Nach Zugabe von 1,2 ml N-Methylmorpholin wird während 48 Stunden bei Zimmertemperatur gerührt, wobei langsam Kristallisation einsetzt. Die Mischung wird während 4 Tagen stehen gelassen, der Niederschlag wird abgenutscht, mit Aethylacetat gewaschen und im Vakuum getrocknet. Man erhält 3,52 g N - Allyl - N - Methylmorpholin - Salz der 2 - (2 - Amino - 4 - thiazolyl) - 2 - [[(Z) - (t - butoxycarbonyl)methoxy] - imino] - essigsäure.

Das rohe Salz wird in 100 ml trockenem Acetonitril suspendiert. 0,49 ml N-Methylmorpholin und 3,32 g 2,2'-Dithiobenzothiazol werden nacheinander unter Rühren hinzugefügt und die erhaltene Suspension auf 5° abgekühlt. Innerhalb 4 Stunden wird nun eine Lösung von 2,5 ml Triäthylphosphit in 30 ml trockenem Acetonitril zugetropft. Nach weiterem Rühren während 30 Minuten wird die erhaltene gelbe Suspension auf −10° abgekühlt. Die Kristalle werden abgenutscht und mit kaltem Acetonitril und Aether gewaschen. Man erhält 1,9 g 2 - (2 - Amino - 4 - thiazolyl) - 2 - [[(Z) - (t - butoxycarbonyl)methoxy] - imino] - essigsäure - 2 - benzthiazolylthioester vom Schmelzpunkt 142—143°C (Zers.).

54,2 g (180 mMol) 2 - (2 - Amino - 4 - thiazolyl) - 2 - [[(Z) - (t - butoxycarbonyl)methoxy] - imino] - essigsäure werden in 1,4 l trockenem Acetonitril dispergiert. 29,6 ml (270 mMol) N-Methylmorpholin werden unter Rühren hinzugefügt, gefolgt von 72,1 g (216 mMol) 2,2'-Dithiobisbenzothiazol. Die erhaltene Suspension wird auf 0°C abgekühlt. Innerhalb 4 1/2 Stunden wird nun eine Lösung von 53,8 ml (314 mMol) Triäthylphosphit in 350 ml trockenem Acetonitril hinzugefügt. Nach weiterem Rühren während 30 Minuten wird die erhaltene gelbe Suspension auf −10°C abgekühlt. Die Kristalle werden abfiltriert und mit kaltem Acetonitril und Aether gewaschen. Man erhält 59,7 g (73,6%) 2 - (2 - Amino - 4 - thiazolyl) - 2 - [[(Z) - (t - butoxycarbonyl) - methoxy] - imino] - essigsäure - 2 - benzthiazolylthioester.

Elementaranalyse berechnet für $C_{18}H_{18}N_4O_4S_3$ (450, 561):

C 47,99, H 4,03, N 12,44, S 21,35%

gefunden

C 47,88, H 4,34, N 12,34, S 21,02%.

Das als Ausgangsmaterial verwendete (3S,4S) - 3 - Amino - 4 - carbamoyloxymethyl - 2 - oxo - 1 - azetidinsulfonsäure - Natriumsalz kann wie folgt hergestellt werden:

a) Zu einer bei Raumtemperatur gerührten Lösung von 0,9 g (5,4 mMol) 2,4-Dimethoxybenzylamin in 100 ml Methylenchlorid gibt man 3 g Molekularisieb 4Å und nach 20 Minuten 0,7 g (5,4 mMol) Isopropyliden-L-glyceraldehyd und 5 g wasserfreies Magnesiumsulfat. Anschliessend rührt man noch während 1 Stunde bei Raumtemperatur. Die erhaltene organische Lösung von Isopropyliden - L - glyceraldehyd - (2,4 - dimethoxybenzyl)imin wird unter Argon auf −20°C gekühlt und unter Rühren mit 0,88 ml (5,4 mMol) Triäthylamin versetzt. Dann wird innerhalb 1 Stunde eine Lösung von 1,25 g (5,6 mMol) Phthaloylglycylchlorid in 20 ml trockenem Methylenchlorid zugetropft und anschliessend über Nacht bei Raumtemperatur weiter gerührt. Die Reaktionsmischung wird dreimal mit je 100 ml Wasser und mit 100 ml Kochsalzlösung gewaschen und über Natriumsulfat getrocknet. Man dampft ein und chromatographiert den Rückstand an Kieselgel (230—400 mesh) unter Eluieren mit Hexan/Essigester (1:1). Man erhält 1,77 g (70%) N - [(3S,4S) - cis - 1 - (2,4 - Dimethoxybenzyl) - 4 - [(R) - 2,2 - dimethyl - 1,3 - dioxolan - 4 - yl] - 2 - oxo - 3 - azetidinyl] - phthalimid als Schaum; $[\alpha]_D = +41°$ (c = 0,8 in Chloroform); MS: 466 ($M^+$).

b) Man versetzt eine Lösung von 149,3 g (0,32 Mol) N - [(3S,4S) - cis - 1 - (2,4 - Dimethoxybenzyl) - 4 - [(R) - 2,2 - dimethyl - 1,3 - dioxolan - 4 - yl] - 2 - oxo - 3 - azetidinyl]phthalimid in 2,5 l Methylenchlorid mit 34 ml (0,64 Mol) Methylhydrazin. Man rührt über Nacht bei 28°C, filtriert vom ausgefallenen Material ab und dampft das Filtrat unter vermindertem Druck ein. Man nimmt den Rückstand in 1,2 l Essigester auf und filtriert die erhaltene Suspension. Das Filtrat wird dreimal mit je 500 ml Wasser und mit 500 ml Kochsalzlösung gewaschen und über Natriumsulfat getrocknet. Nach Eindampfen des Lösungsmittels erhält man 104,3 g (86,8%) rohes (3S,4S) - cis - 3 - Amino - 1 - (2,4 - dimethoxybenzyl) - 4 - [(R) - 2,2 - dimethyl - 1,3 - dioxolan - 4 - yl] - 2 - azetidinon, das ohne weitere Reinigung in die nächste Stufe eingesetzt wird.

c) Man versetzt eine gerührte Lösung von 104 g (3,0 Mol) (3S,4S) - cis - 3 - Amino - 1 - (2,4 - dimethoxybenzyl) - 4 - [(R) - 2,2 - dimethyl - 1,3 - dioxolan - 4 - yl] - 2 - azetidinon und 104 ml (1,2 Mol) Butylenoxid in 1,5 l Methylenchlorid tropfenweise mit 57,6 ml (0,4 Mol) Carbobenzoxychlorid, rührt

während 1 Stunde und dampft anschliessend unter vermindertem Druck ein. Das erhaltene rohe Material wird mit 2 l trockenem Aether behandelt, wobei man ein kristallines Material erhält. Man erhält 122,6 g (84%) Benzyl - (3S,4S) - cis - 1 - (2,4 - dimethoxybenzyl) - 4 - [(R) - 2,2 - dimethyl - 1,3 - dioxolan - 4 - yl] - 2 - oxo - 3 - azetidincarbamat vom Schmelzpunkt 115—116°C; $[\alpha]_D = +48°$ (c = 0,3 in Methanol).

d) Eine Lösung von 160 g (0,34 Mol) Benzyl - (3S,4S) - cis - 1 - (2,4 - dimethoxybenzyl) - 4 - [(R) - 2,2 - dimethyl - 1,3 - dioxolan - 4 - yl] - 2 - oxo - 2 - azetidincarbamat in 1000 ml Tetrahydrofuran und 400 ml Wasser wird in Gegenwart von 8 g p-Toluolsulfonsäure bei 60°C über Nacht gerührt. Die Reaktionsmischung wird mit ges. $NaHCO_3$ neutralisiert und das Tetrahydrofuran wird eingedampft. Die wässrige Lösung wird dann mit 2 l Essigester extrahiert. Nach Trocknung über $Na_2SO_4$ und Eindampfen erhält man 142 g (97,2%) reines Benzyl - (3S,4S) - cis - 4 - [(R) - 1,2 - dihydroxyäthyl] - 1 - (2,4 - dimethoxybenzyl) - 2 - oxo - 3 - azetidincarbamat, Smp. 177—178°C (MeOH).

e) Man versetzt eine Lösung von 142 g (0,33 Mol) Benzyl - (3S,4S) - cis - 4 - [(R) - 1,2 - dihydroxyäthyl] - 1 - (2,4 - dimethoxybenzyl) - 2 - oxo - 3 - azetidincarbamat in 1000 ml Tetrahydrofuran unter Rühren tropfenweise mit einer Lösung von 76,8 g (0,359 Mol) Natriummetaperjodat in 600 ml Wasser. Man rührt während 1 Stunde, filtriert und dampft das Filtrat unter vermindertem Druck ein. Man nimmt den Rückstand in 400 ml Essigester auf und wäscht zweimal mit je 100 ml Wasser und mit 50 ml Kochsalzlösung. Nach Trocknen und Eindampfen erhält man 105 g (87,8%) reines Benzyl - (3S,4S) - cis - 1 - (2,4 - dimethoxybenzyl) - 4 - formyl - 2 - oxo - 3 - azetidincarbamat von Schmelzpunkt 145—147°C (aus Essigester/Hexan); $[\alpha]_D = +13,7°$ (c = 1, $CHCl_3$).

f) 4,27 g (113 mMol) Natriumborhydrid werden in 1,6 l abs. Aethanol gelöst und auf 0°C abgekühlt. Diese Lösung wird mit einer Lösung von 90 g (226 mMol) Benzyl - (3S,4S) - cis - 1 - (2,4 - dimethoxybenzyl) - 4 - formyl - 2 - oxo - 3 - azetidincarbamat in 720 ml Aethanol-Tetrahydrofuran (1:1) tropfenweise versetzt. Das Reaktionsgemisch wird 2 Stunden bei 0°C gerührt und anschliessend mit 350 ml gesättigter wässriger Natriumsulfatlösung behandelt und 45 Minuten gerührt. Nach dem Filtrieren und Abdampfen des Lösungsmittels wird der Rückstand in 1,5 l Aethylacetat aufgenommen und bis zu neutraler Reaktion gewaschen. Nach dem Trocknen über Natriumsulfat und teilweisem Eindampfen erhält man kristallines (3S,4S) - cis - 3 - Benzyloxycarboxamido - 1 - (2,4 - dimethoxybenzyl) - 4 - hydroxymethyl - 2 - azetidinon in Form von 72,2 g farblosen Kristallen (79,6%) vom Schmelzpunkt 138°C, $[\alpha]_D = +41,6°$ (c = 1 in Methanol).

Elementaranalyse berechnet für $C_{21}H_{24}N_2O_6$:
C 62,99, H 6,04, N 7,00%
gefunden
C 62,76, H 6,09, N 6,96%
IR (KBr, $cm^{-1}$):
1718, 1698, 1615, 1589
NMR ($CDCl_3$, ppm):
2,45 (1H, dd, OH), 3.55—3.75 (3H, broad, CH—$CH_2$—), 3.79 (6H, s, 2×$OCH_3$), 4,35 (2H, s, N—$CH_2$), 5,08 (2H, s, Ø—$CH_2$), 5,11 (1H, dd, 5 und 9 Hz, $H_3$), 6,06 (1H, d, 9Hz, NH), 6,43 (2H, m, Ar), 7,15 (1H, m, Ar), 7.31 (5H, m, $C_6H_5$)
MS:
292 (M—BzOH)

g) Eine Lösung von 30 g (74,9 mMol) (3S,4S) - cis - 3 - Benzyloxycarboxamido - 1 - (2,4 - dimethoxybenzyl) - 4 - hydroxymethyl - 2 - azetidinon in 600 ml Methylenchlorid werden bei 0—5°C mit 21,22 g Chlorsulfonyl-isocyanat (2 Aequivalenten) behandelt. Nach 15 Minuten wird das Reaktionsgemisch tropfenweise zu einer wässrigen, auf 5°C gekühlten Lösung von 20,9 g (2,7 Aequivalenten) Natriumsulfit gegeben. Das Reaktionsgemisch wird 2 Stunden gerührt, anschliessend mit Methylenchlorid verdünnt und die organische Phase abgetrennt, mit wässriger Natriumchloridlösung gewaschen und 12 Stunden über Natriumsulfate getrocknet. Die organische Phase wird anschliessend mit Magnesiumsulfat behandelt und weitere 2 Stunden gerührt. Nach dem Filtrieren und Abdampfen des Lösungsmittels wird der Rückstand mit Aether behandelt, die erhaltenen Kristalle werden filtriert und mit Aether gewaschen. Man erhält 32,6 g (97%) (3S,4S) - cis - 3 - Benzyloxycarboxamido - 4 - carbamoyloxymethyl - 1 - (2,4 - dimethoxybenzyl) - 2 - azetidinon vom Schmelzpunkt 178—179°C, $[\alpha]_D = +84,7°$ (c = 0,8 in Chloroform).

Elementaranalyse: berechnet für $C_{22}H_{25}N_3O_7$:
C 59,59, H 5,68, N 9,49%
gefunden
C 59,17, H 5,69, N 9,37%
IR (KBr, $cm^{-1}$):
1761, 1708, 1618, 1587

h) Eine Suspension von 11,9 g (26,8 mMol) (3S,4S) - cis - 3 - Benzyloxycarboxamido - 4 - carbamoyloxymethyl - 1 - (2,4 - dimethoxybenzyl) - 2 - azetidinon, 14,5 g (53,5 mMol) Kaliumperoxidisulfat, 13,98 g (80,3 mMol) Dikaliumhydrogenphosphat und 1,33 g (5,36 mMol) Kupfersulfat ($5H_2O$) in 270 ml Acetonitril und 130 ml Wasser werden in einer Argonatmosphäre 3 1/2 Stunden auf 95°C erhitzt, bei einem pH-Wert zwischen 6,5 und 7,0 (zeitweilige Zugabe von 10 g Dikaliumhydrogensulfat). Nach dem Erkalten und Filtrieren wird die wässrige Phase verworfen und die organische Phase eingedampft. Der Rückstand wird in Aethylacetat aufgenommen und mit Wasser und Kochsalzlösung

gewaschen. Nach dem Trocknen über Natriumsulfat, Filtration und Abdampfen des Lösungsmittels wird der Rückstand in Aether aufgenommen und filtriert. Die rohen Kristalle (8,9 g) werden auf $SiO_2$ chromatographiert (300 g, 40—63 µm, Chloroform:Methanol:Aethylacetat 85:10:5). Man erhält 5,5 g (70%) (3S,4S) - cis - 3 - Benzyloxycarboxamido - 4 - carbamoyloxymethyl - 2 - azetidinon als farblose Kristalle, $[\alpha]_D$ = +61,2° (c = 1 in Methanol). Schmelzpunkt 193—195°C.

Elementaranalyse: berechnet für $C_{13}H_{15}N_3O_5$:
C 53,24, H 5,16, N, 14,33%
gefunden
C 53,40, H 5,24, N 14,35%

IR (KBr, $cm^{-1}$):
3414, 3315, 1757, 1701, 1610, 1540, 1498

NMR (DMSO, ppm):
3,31—4,06 (3H, m, CH—$CH_2$—), 4,95 (1H, dd, 4,5 und 9Hz, $H_3$), 5,06 (2H, s, Ø—$CH_2$), 6,53 (2H, broad, $NH_2$), 7,35 (5H, s, $C_6H_5$), 7,95 (1H, d, 9Hz, $CH_3$—NH—CO), 8,35 (1H, s, NH—CO)

MS (Cl mit $NH_3$):
251 $(M+H)^+$ —CONH

i) 5,4 g (18,4 mMol) (3S,4S) - cis - 3 - Benzyloxycarboxamido - 4 - carbamoyloxymethyl - 2 - azetidinon in 200 ml abs. Dioxan werden bei Zimmertemperatur mit 4,3 g (1,3 Aequivalenten) Pyridin-Schwefeltrioxid-Komplex behandelt. Die erhaltene Suspension wird 3 Stunden gerührt, anschliessend mit weiteren 0,99 g (0,3 Aequivalenten) Pyridin-Schwefeldioxid-Komplex behandelt und das Reaktionsgemisch eine weitere Stunde gerührt. Nach Zugabe weiterer 1,37 g (0,4 Aequivalenten) Pyridin-Schwefeltrioxid-Komplex und weiterem 2-stündigen Rühren wird das Lösungsmittel teilweise unter vermindertem Druck entfernt und der Rückstand mit 110 ml gesättigter wässriger Natriumbicarbonatlösung behandelt. Die erhaltene, braune Lösung wird 12 Stunden im Kühlschrank stehen gelassen und die erhaltenen Kristalle werden abfiltriert. Die Mutterlauge wird chromatographiert (MCI Gel, Wasser-Aethanol 1:1 bis 9:1). Nach dem Lyophilisieren erhält man 3,5 g (49%) (3S,4S) - cis - 3 - Benzyloxycarboxamido - 4 - carbamoyloxymethyl - 2 - azetidinon - 1 - sulfonsäure - Natriumsalz als ein farbloses Pulver, $[\alpha]_D$ = +29,6° (c = 0,5 in Wasser).

Elementaranalyse: berechnet für $C_{13}H_{14}N_3O_8SNa$:
C 39,50, H 3,57, N 10,63%
gefunden
C 39,41, H 3,45, N 10,36%

IR (KBr, $cm^{-1}$):
1798, 1758, 1739, 1693, 1584, 1547

NMR (DMSO, ppm):
3,9—4,4 (3H, CH—$CH_2$), 4,9 (dd, 1H, NH—CH), 5,1 (s, 2H, Ø—$CH_2$), 6,4 (2H, broad, $NH_2$), 7,4 (5H, s, $C_6H_5$), 8,0 (1H, d, NH)

k) 3,065 g (7,75 mMol) (3R,4S) - cis - Benzyloxycarboxamido - 4 - carbamoyloxymethyl - 2 - azetidinon - 1 - sulfonsäure - Natriumsalz werden in 180 ml abs. Methanol gelöst und 1 Stunde in Gegenwart von 1,5 g 10% Palladiumkohle hydriert. Der Katalysator wird durch Filtration entfernt und die erhaltene Lösung eingedampft. Man erhält 2,02 g (100%) (3S,4S) - cis - 3 - Amino - 4 - carbamoyloxymethyl - 2 - oxo - 1 - azetidin - sulfonsäure - Natriumsalz.

IR (KBr, $cm^{-1}$):
3444, 3207, 1754, 1725, 1611, 1249.

**Patentansprüche**

1. Verfahren zur Herstellung von Carbonsäuren der allgemeinen Formel

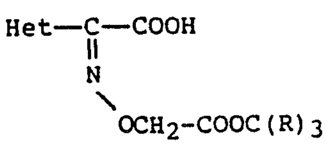

IV a

in der Het einen ggfs. aminosubstituierten 5- oder 6-gliedrigen, aromatischen, 1 oder 2 Stickstoffatome, ggfs. auch ein Sauerstoff- oder Schwefelatom enthaltenden Heterocyclus und R $C_{1-3}$-Alkyl bedeutet, und die Gruppe $=NOCH_2COOC(R)_3$ mindestens teilweise in syn-Form vorliegt, dadurch gekennzeichnet, dass man in einem Ester der allgemeinen Formel

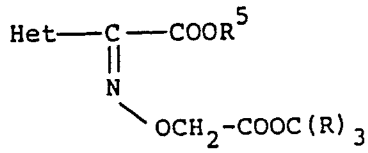

X

in der Het und R die oben gegebene Bedeutung haben und $R^5$ Allyl darstellt, und die Gruppe $=NOCH_2COOC(R)_3$ mindestens teilweise in syn-Form vorliegt,

die Allylgruppe $R^5$ mit Hilfe einer Palladiumverbindung in Gegenwart von Triphenylphosphin oder eines Tri(niederen Alkyl)phosphits und ferner in Gegenwart eines Alkalimetallalkanoats oder einer organischen Base katalytisch abspaltet.

2. Verfahren nach Anspruch 1, dadurch gekennzeichnet, dass man als Palladiumverbindung Palladiumkohle, einen palladiumorganischen Komplex mit Triphenylphosphin oder einem Tri(niederen Alkyl)phosphit oder ein Palladiumsalz mit einer Halogenwasserstoffsäure oder einer niederen Alkancarbonsäure verwendet.

3. Verfahren nach Anspruch 2, dadurch gekennzeichnet, dass man als Palladiumverbindung Palladiumkohle, Palladiumchlorid oder Palladiumacetat verwendet.

4. Verfahren nach einem der Ansprüche 1—3, dadurch gekennzeichnet, dass man als Tri(niederes Alkyl)phosphit Triäthylphosphit verwendet.

5. Verfahren nach einem der Ansprüche 1—4, dadurch gekennzeichnet, dass man die Spaltung in Gegenwart einer organischen Base durchführt.

6. Verfahren nach Anspruch 5, dadurch gekennzeichnet, dass man als organische Base N-Methylmorpholin oder Triäthylamin verwendet.

7. Verfahren nach einem der Ansprüche 1—6, dadurch gekennzeichnet, dass R Methyl bedeutet.

8. Verfahren nach einem der Ansprüche 1—7, dadurch gekennzeichnet, dass Het 2-Amino-4-thiazolyl bedeutet.

9. Verfahren nach einem der Ansprüche 1—8, dadurch gekennzeichnet, dass man eine erhaltene Carbonsäure der allgemeinen Formel

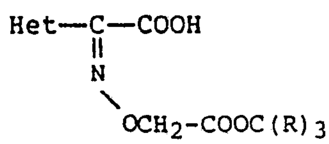

IV a

in der Het und R die in Anspruch 1 angegebene Bedeutung haben, und die Gruppe $=NOCH_2COOC(R)_3$ mindestens teilweise in syn-Form vorliegt,

mit Dithio-bis-benzthiazol in Gegenwart eines Tri(niederen Alkyl)phosphits und einer Base oder in Gegenwart von Triphenylphosphin in einen Benzthiazolthioester der allgemeinen Formel

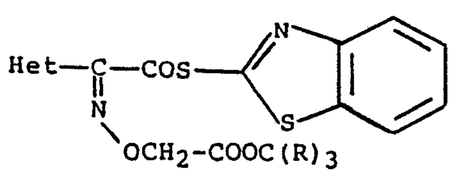

III

in der R und Het die in Anspruch 1 angegebene Bedeutung haben, und die Gruppe $—NOCH_2COOC(R)_3$ mindestens teilweise in syn-Form vorliegt,

überführt.

10. Verfahren nach Anspruch 9, dadurch gekennzeichnet, dass man die Umsetzung mit Dithio-bis-benzthiazol in Gegenwart eines Tri(niederen Alkyl)phosphits durchführt.

11. Verfahren nach Anspruch 10, dadurch gekennzeichnet, dass man als Tri(niederes Alkyli)phosphit Triäthylphosphit verwendet.

12. Verfahren nach Anspruch 10 oder 11, dadurch gekennzeichnet, dass man als Base eine organische Base verwendet.

13. Verfahren nach Anspruch 12, dadurch gekennzeichnet, das man als organische Base N-Methylmorpholin verwendet.

14. Verfahren nach einem der Ansprüche 9—13, dadurch gekennzeichnet, dass man einen erhaltenen Benzthiazolthioester der allgemeinen Formel

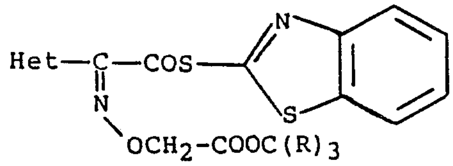

III

in der Het und R die in Anspruch 1 angegebene Bedeutung haben, und die Gruppe $=NOCH_2COOC(R)_3$ teilweise in syn-Form vorliegt,
durch Umsetzen mit einer in racemischer Form oder in Form des 3S-Enantiomeren vorliegenden Verbindung der allgemeinen Formel

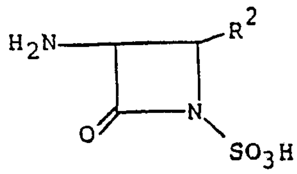

II

worin $R^2$ Wasserstoff, niederes Alkyl, niederes Alkenyl, niederes Alkinyl, niederes Alkoxycarbonyl, niederes Alkanoyloxy-niederes Alkyl, niederes Alkoxycarbonyl-niederes Alkenyl, Hydroxyiminomethyl, niederes Alkoxyiminomethyl, Carbamoyl, Carbamoyl-niederes Alkenyl oder Carbamoyloxy-niederes Alkyl bedeutet, wobei die mit nieder bezeichneten Gruppen bis zu 7 Kohlenstoffatome besitzen,
in eine in racemischer Form oder in Form des 3S-Enantiomeren vorliegende Verbindung der allgemeinen Formel

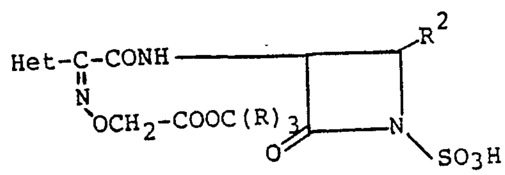

I

worin Het und R die in Anspruch 1 angegebene und $R^2$ obige Bedetung besitzen, und die Gruppe $=NOCH_2COOC(R)_3$ mindestens teilweise in syn-Form vorliegt, überführt, erwünschtenfalls die Gruppe $-CH_2-COOC(R)_3$
in Carboxymethyl umwandelt und erwünschtenfalls ein erhaltenes Produkt in ein pharmazeutisch verträgliches Salz überführt.

15. Verfahren nach Anspruch 14, dadurch gekennzeichnet, dass man die Umsetzung der Verbindungen der Formeln II und III in Gegenwart eines organischen Amins, wie Triäthylamin, durchführt.

16. Verfahren nach Anspruch 14 oder 15, dadurch gekennzeichnet, dass man eine optisch einheitliche 3,4-cis-Verbindung (insbesondere eine 3S,4S-Verbindung) der Formel II einsetzt.

17. Verfahren nach einem der Ansprüche 14—16, dadurch gekennzeichnet, dass man als Thioester der Formel III 2 - (2 - Amino - 4 - thiazolyl) - 2 - [[(Z) - 1 - (t - butoxycarbonyl)methoxy] - imino]essigsäure - 2 - benzthiazolyl - thioester verwendet und im Reaktionsprodukt die Carboxygruppe freisetzt.

18. Verfahren nach Anspruch 17, dadurch gekennzeichnet, das man eine t-Butoxycarbonylgruppe durch Behandeln mit Salzsäure oder Trifluoressigsäure in Carboxy umwandelt.

19. Verfahren nach einem der Ansprüche 14—18, dadurch gekennzeichnet, dass man (Z) - 2 - (2 - Amino - 4 - thiazolyl) - 2 - [[1 - [t - butoxycarbonyl]methoxy]imino]essigsäure - 2 - benzthiazolyl - thioester mit (3S,4S) - 3 - Amino - 4 - carbamoyloxymethyl - 2 - oxo - 1 - azetidinsulfonsäure umsetzt und im Reaktionsprodukt die Carboxygruppe durch Behandeln mit einer starken organischen Säure freisetzt.

20. Verbindungen der allgemeinene Formel

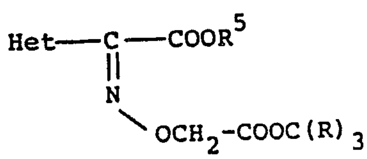

X

in der Het und R die in Anspruch 1 angegebene Bedeutung haben, $R^5$ Allyl bedeutet, und die Gruppe $=NOCH_2COOC(R)_3$ mindestens teilweise in syn-Form vorliegt.

21. Verbindungen gemäss Anspruch 20, worin R Methyl bedeutet.

22. Verbindungen gemäss Anspruch 20 oder 21, worin Het 2-Amino-4-thiazolyl bedeutet.

23.2 - (2 - Amino - 4 - thiazolyl) - 2 - [[(Z) - (t - butoxycarbonyl)methoxy]imino]essigsäureallylester.

**Revendications**

1. Procédé de préparation d'acides carboxyliques de formule générale

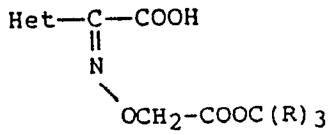

IV a

où Het représente un hétérocycle aromatique à 5 ou 6 chaînons éventuellement amino-substitué contenant 1 ou 2 atomes d'azote, et éventuellement également un atome d'oxygène ou de soufre et R représente un alcoyle en C1 à C3, et le groupe $=NOCH_2\ COOC(R)_3$ se présente au moins en partie sous forme syn,

caractérisé en ce que dans un ester de formule générale

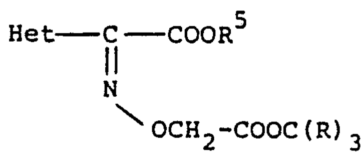

X

où Het et R ont la signification donnée ci-dessus et R5 représente un allyle, et le groupe $=NOCH_2\ COOC(R)_3$ se présente au moins en partie sous forme syn,

on sépare par catalyse le groupe allyle R5 à l'aide d'un composé de palladium en présence de triphénylphosphine ou d'un phosphite de tri(alcoyle inférieur) et en outre en présence d'un alcanoate de métal alcalin ou d'une base organique.

2. Procédé selon la revendication 1, caractérisé en ce qu'on utilise comme composé de palladium le palladium-charbon, un complexe organique de palladium avec la triphénylphosphine ou un tri(alcoyle inférieur)phosphite ou un sel de palladium avec un acide halohydrique ou un acide alcane inférieur-carboxylique.

3. Procédé selon la revendication 2, caractérisé en ce qu'on utilise comme composé de palladium le palladium-charbon, le chlorure de palladium ou l'acétate de palladium.

4. Procédé selon l'une des revendications 1—3, caractérisé en ce qu'on utilise comme tri(alcoyle inférieur)phosphite le triéthylphosphite.

5. Procédé selon l'une des revendications 1—4, caractérisé en ce qu'on effectue la séparation en présence d'une base organique.

6. Procédé selon la revendication 5, caractérisé en ce qu'on utilise comme base organique la N-méthylmorpholine ou la triéthylamine.

7. Procédé selon l'une des revendications 1—6, caractérisé en ce que R représente un méthyle.

8. Procédé selon l'une des revendications 1—7, caractérisé en ce que Het représente le 2-amino-4-thiazolyle.

9. Procédé selon l'une des revendications 1—8, caractérisé en ce qu'on transforme un acide carboxylique obtenu de formule générale

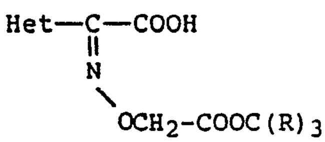

IV a

où Het et R ont la signification donnée dans la revendication 1 et le groupe $=NOCH_2COOC(R_3)$ se présente au moins en partie sous forme syn,

avec du dithio-bis-benzthiazole en présence d'un tri(alcoyle inférieur)phosphite et d'une base ou en présence de triphénylphosphine en un benzthiazolthioester de formule générale

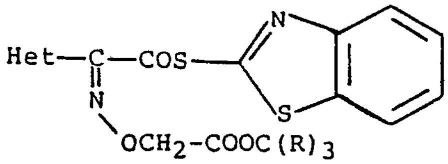

III

où R et Het ont la signification donnée dans la revendication 1 et le groupe $=NOCH_2$ $COOC(R)_3$ se présente au moins en partie sous forme syn.

10. Procédé selon la revendication 9, caractérisé en ce qu'on conduit la réaction avec du dithio-bis-benzthiazole en présence d'un tri(alcoyle inférieur)phosphite.

11. Procédé selon la revendication 10, caractérisé en ce qu'on utilise comme tri(alcoyle inférieur)phosphite. le triéthylphosphite.

12. Procédé selon la revendication 10 ou 11, caractérisé en ce qu'on utilise comme base une base organique.

13. Procédé selon la revendication 12, caractérisé en ce qu'on utilise comme base organique la N-méthylmorpholine.

14. Procédé selon l'une des revendications 9—13, caractérisé en ce qu'on transforme un benzthiazolthioester obtenu de formule générale

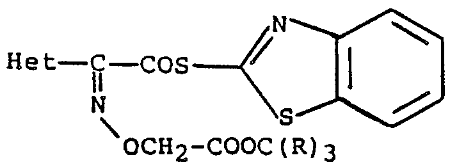

III

où Het et R ont la signification donnée dans la revendication 1, et le groupe $=NOCH_2$ $COOC(R)_3$ se présente en partie sous forme syn,

par réaction avec un composé, présent sous forme racémique ou sous la forme de l'énantiomère 3S, de formule générale

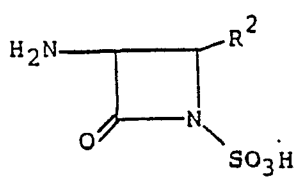

II

où R2 représente un hydrogène, alcoyle inférieur, alcényle inférieur, alcynyle inférieur, alcoxy inférieur-carbonyle, alcanoyloxy inférieur-alcoyle inférieur, alcoxy inférieur-carbonyl-alcényle inférieur, hydroxyiminométhyle, alcoxy inférieur-iminométhyle, carbamoyle, carbamoyl-alcényle inférieur ou carbamoyloxy-alcoyle inférieur, où les groupes désignés comme inférieurs possèdent jusqu'à 7 atomes de carbone, en un composé, présent sous forme racémique ou sous la forme de l'énantiomère 3S, de formule générale

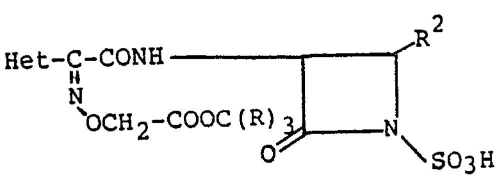

I

où Het et R ont la signification donnée dans la revendication 1 et R2 a la signification donnée ci-dessus, et le groupe $—NOCH_2$ $COOC(R_3)$ se présente au moins en partie sous forme syn,

si on le désire en ce qu'on transforme le groupe $—CH_2—COOC(R)_3$ en carboxyméthyle et si on le désire en ce qu'on transforme un produit obtenu en un sel pharmaceutiquement acceptable.

15. Procédé selon la revendication 14, caractérisé en ce qu'on conduit la réaction des composés de formules II et III en présence d'une amine organique, comme la triéthylamine.

16. Procédé selon les revendications 14 ou 15, caractérisé en ce qu'on utilise un composé 3,4-cis (en particulier un composé 3S,4S) optiquement uniforme de formule II.

17. Procédé selon l'une des revendications 14—16, caractérisé en ce qu'on utilise comme thioester de formule III le 2 - benzthiazolyl - thioester de l'acide 2 - (2 - amino - 4 - thiazolyl) - 2 - [[(Z) - 1 - (t - butoxycarbonyl) - méthoxy] - imino]acétique et en ce qu'on libère le groupe carboxy dans le produit de la réaction

18. Procédé selon la revendication 17, caractérisé en ce qu'on transforme un groupe t-butoxycarbonyle par traitement avec de l'acide chlorhydrique ou de l'acide trifluoracétique en carboxy.

19. Procédé selon l'une des revendications 14—18, caractérisé en ce qu'on fait réagir le 2-benzthiazolylthioester de l'acide (Z) - 2 - (2 - amino - 4 - thiazolyl) - 2 - [[1 - [t - butoxycarbonyl]-méthoxy]imino]acétique avec l'acide (3S,4S) - 3 - amino - 4 - carbamoyloxyméthyl - 2 - oxo - 1 - azétidinesulfonique et en ce que dans le produit de la réaction ou libère le groupe carboxy par traitement avec un acide organique fort.

20. Composés de formule générale

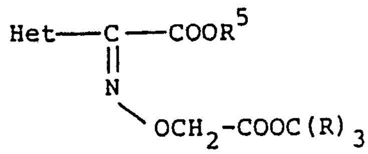

X

où Het et R ont la signification donnée dans la revendication 1, R5 représente un allyle et le groupe $=NOCH_2\ COOC(R)_3$ se présente au moins en partie sous forme syn.

21. Composés selon la revendication 20, où R représente un méthyle.

22. Composés selon la revendication 20 ou 21, où Het représente un 2-amino-4-thiazolyle.

23. Allylester de l'acide 2 - (2 - amino - 4 - thiazolyl) - 2 - [[(Z) - (t - butoxycarbonyl)méthoxy]imino]-acétique.

**Claims**

1. A process for the manufacture of carboxylic acids of the general formula

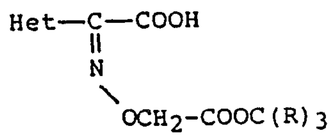

IV a

in which Het signifies an optionally amino-substituted 5- or 6-membered aromatic heterocycle containing 1 or 2 nitrogen atoms and optionally also an oxygen or sulphur atom and R signifies $C_{1-3}$-alkyl, and the group $=NOCH_2COOC(R)_3$ is present at least partially in the syn-form,

characterized by cleaving off catalytically the allyl group $R^5$ in an ester of the general formula

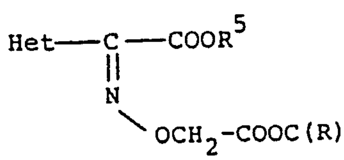

X

in which Het and R have the significance given above and $R^5$ represents allyl, and the group $=NOCH_2COOC(R)_3$ is present at least partially in the syn-form,

with the aid of a palladium compound in the presence of triphenylphosphine or a tri(lower alkyl)phosphite and in addition in the presence of an alkali metal alkanoate or an organic base.

2. A process according to claim 1, characterized in that palladium/carbon, a palladium-organic complex with triphenylphosphine or a tri(lower alkyl)phosphite or a palladium salt with a hydrohalic acid or a lower alkanecarboxylic acid is used as the palladium compound.

3. A process according to claim 2, characterized in that palladium/carbon, palladium chloride or palladium acetate is used as the palladium compound.

4. A process according to any one of claims 1—3, characterized in that triethylphosphite is used as the tri(lower alkyl)phosphite.

5. A process according to any one of claims 1—4, characterized in that the cleavage is carried out in the presence of an organic base.

6. A process according to claim 5, characterized in that N-methyl-morpholine or triethylamine is used as the organic base.

7. A process according to any one of claims 1—6, characterized in that R signifies methyl.

8. A process according to any one of claims 1—7, characterized in that Het signifies 2-amino-4-thiazolyl.

9. A process according to any one of claims 1—8, characterized in that an obtained carboxylic acid of the general formula

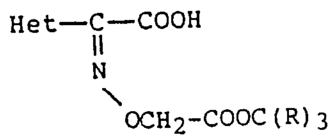

IV a

in which Het and R have the significance given in claim 1 and the group $=NOCH_2COOC(R)_3$ is present at least partially in the syn-form,

is converted into a benzthiazole thioester of the general formula

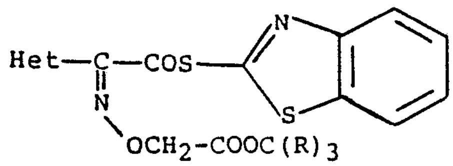

III

in which R and Het have the significance given in claim 1 and the group $—NOCH_2COOC(R)_3$ is present at least partially in the syn-form,

with dithio-bis-benzthiazole in the presence of a tri(lower alkyl)phosphite and a base or in the presence of triphenylphosphine.

10. A process according to claim 9, characterized in that the reaction with dithio-bis-benzthiazole is carried out in the presence of a tri(lower alkyl)phosphite.

11. A process according to claim 10, characterized in that triethylphosphite is used as the tri(lower alkyl)phosphite.

12. A process according to claim 10 or 11, characterized in that an organic base is used as the base.

13. A process according to claim 12, characterized in that N-methylmorpholine is used as the organic base.

14. A process according to any one of claims 9—13, characterized in that an obtained benzthiazole thioester of the general formula

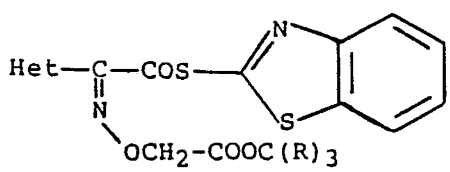

III

in which Het and R have the significance given in claim 1 and the group $=NOCH_2COOC(R)_3$ is present partially in the syn-form,

is converted by reaction with a compound, which is present in racemiound, which is present in racemic form or in the form of the 3S-enantiomer, of the general formula

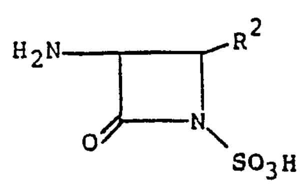

II

wherein $R^2$ signifies hydrogen, lower alkyl, lower alkenyl, lower alkynyl, lower alkoxycarbonyl, lower alkanoyloxy-lower alkyl, lower alkoxycarbonyl-lower alkenyl, hydroxyiminomethyl, lower alkoxyiminomethyl, carbamoyl, carbamoyl-lower alkenyl or carbamoyloxy-lower alkyl, whereby the groups denoted by lower have up to 7 carbon atoms,

into a compound, which is present in racemic form or in the form of the 3S-enantiomer, of the general formula

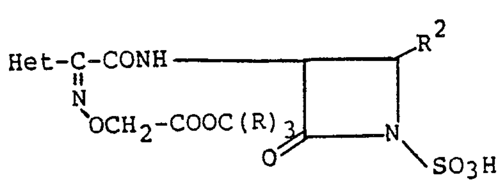

I

wherein Het and R have the significance given in claim 1 and $R^2$ has the above significance and the group $=NOCH_2COOC(R)_3$ is present at least partially in the syn form,

if desired converting the group $—CH_2COOC(R)_3$ into carboxymethyl and, if desired, converting a product obtained into a pharmaceutically compatible salt.

15. A process according to claim 14, characterized in that the reaction of the compounds of formulae II and III is carried out in the presence of an organic amine such as triethylamine.

16. A process according to claim 14 or 15, characterized in that an optically uniform 3,4-cis compound (especially a 3S,4S compound) of formula II is used.

17. A process according to any one of claims 14—16, characterized in that 2 - (2 - amino - 4 - thiazolyl) - 2 - [[(Z) - 1 - (t - butoxycarbonyl)methoxy]imino]acetic acid 2-benzthiazolyl thioester is used as the thioester of formula III and the carboxy group is liberated in the reaction product.

18. A process according to claim 17, characterized in that a t-butoxycarbonyl group is converted into carboxy by treatment with hydrochloric acid or trifluoroacetic acid.

19. A process according to any one of claims 14—18, characterized in that 2 - (2 - amino - 4 - thiazolyl) - 2 - [[(Z) - 1 - (t - butoxycarbonyl)methoxy]imino]acetic acid 2-benzthiazolyl thioester is reacted with (3S,4S) - 3 - amino - 4 - carbamoyloxymethyl - 2 - oxo - 1 - azetidinesulphonic acid and the carboxy group is liberated in the reaction product by treatment with a strong organic acid.

20. Compounds of the general formula

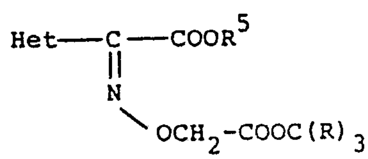

X

in which Het and R have the significance given in claim 1, $R^5$ signifies allyl and the group $=NOCH_2COOC(R)_3$ is present at least partially in the syn-form.

21. Compounds in accordance with claim 20, wherein R signifies methyl.

22. Compounds in accordance with claim 20 or 21, wherein Het signifies 2-amino-4-thiazolyl.

23. 2-(2-Amino-4-thiazolyl)-2-[[(Z)-(t)-butoxycarbonyl)methoxy]imino]acetic acid allyl ester.